# EUROPEAN PATENT SPECIFICATION

(11) **EP 2 090 538 B1**
(45) Date of publication and mention of the grant of the patent: **27.02.2013**
(21) Application number: 08020576.8
(22) Date of filing: 26.11.2008
(51) Int. Cl.: B65H 54/10, B65H 61/00, B65H 63/06, D01H 13/22, G01N 33/36

(54) **Yarn quality measuring instrument and yarn winding machine**
Garnqualitätmessinstrument und Garnwickelmaschine
Instrument de mesure de la qualité de fil et machine de renvideur de fil

(30) Priority: 14.02.2008 JP 2008033845
(43) Date of publication of application: 19.08.2009
(73) Proprietor: Murata Machinery, Ltd., Kyoto 601 (JP)
(72) Inventor: Nakade, Kazuhiko, Kyoto-shi Kyoto (JP); Nakatani, Masatoshi, Kyoto-shi Kyoto (JP); Tsukamoto, Shinichi, Kyoto-shi Kyoto (JP)
(74) Representative: Liedl, Christine

(56) References cited:
- EP-A1- 1 795 477
- EP-A2- 1 024 213
- EP-A2- 1 101 846
- DE-A1- 10 310 178
- JP-A- 2002 348 043
- US-A- 5 592 849

## Description

### Field of the Invention

The present invention relates to a yarn quality measuring instrument, and in particular, to a yarn quality measuring instrument that samples a yarn unevenness signal to detect a yarn defect. The present invention also relates to a yarn winding machine including the yarn quality measuring instrument.

### Background of the Invention

A conventionally known arrangement in a textile machine such as a spinning machine or an automatic winder includes a yarn clearer (yarn quality measuring instrument) that monitors the thickness of a traveling yarn. The spinning machine is configured such that the yarn clearer monitors the thickness of the yarn spun at a constant speed so that when the thickness of the yarn falls continuously out of a predetermined range over at least a predetermined length of the yarn, the yarn clearer determines that a yarn defect has occurred and removes the yarn defect.

In the automatic winder, the length over which the thickness unevenness appears is calculated in accordance with a rotation pulse signal from a winding drum rotationally driven in contact with a winding package.

However, when the automatic winder winds a yarn into a cone-shaped package, technically speaking, a winding speed varies between a larger diameter side and a smaller diameter side in spite of the constant rotation speed of the winding drum. Thus, in the conventional art, when the yarn is wound around the larger diameter side of the package, a yarn speed is high, and the length over which the thickness unevenness appears may be determined by the yarn clearer to be shorter than the actual length. The yarn clearer may thus miss the yarn defect to be otherwise detected, resulting in degraded quality of the package. On the other hand, when the yarn is wound around the smaller diameter side of the package, the yarn speed is low, and the length of the thickness unevenness may be determined by the yarn clearer to be longer than the actual length. The yarn clearer may determine the otherwise tolerable thickness unevenness to be a yarn defect and cuts the corresponding part. This may result in wasted yarns and degraded productivity. In particular, in order to prevent a possible failure to detect a yarn defect when the yarn is wound around the larger diameter side, a stricter value for the tolerable length of the thickness unevenness than necessary needs to be set in the yarn clearer. However, in this case, when the yarn is wound around the smaller diameter side of the package, winding is frequently suspended due to the detection of the otherwise tolerable thickness unevenness as a yarn defect.

Meanwhile, even when the automatic winder winds the yarn into a cheese-shaped package, since a groove formed in the drum for traversing does not have a uniform depth, the winding speed is actually not constant. Furthermore, regardless of whether the package is shaped like a cone or cheese, the drum may be accelerated or decelerated during winding to allow the package to slide with respect to the drum, in order to avoid a critical wind number for ribbon winding. This may also conventionally vary the winding speed. As described above, in the conventional art, the yarn clearer provided in the automatic winder fails to correctly evaluate the length of the yarn defect.

As pointed out in the Unexamined Japanese Patent Application Publication (Tokkai-Hei) No. 2002-348043 relating to a yarn wider that rotationally moves a winding package by friction of a driving roller, the length of a yarn defect obtained by evaluating a pulse from the driving roller is disadvantageously inaccurate for cleaning of the yarn. To solve this problem, the Unexamined Japanese Patent Application Publication (Tokkai-Hei) No. 2002-348043 discloses a yarn winding machine configured to wind a yarn into a package and including a clearer with a traveling time correlater. The yarn winding machine described in the Unexamined Japanese Patent Application Publication (Tokkai-Hei) No. 2002-348043 includes two yarn sensors at respective measurement points. Measured values from the two yarn sensors are evaluated via the traveling time correlater and the yarn speed is checked. The package is configured to be rotated by the friction of the driving roller. A polar wheel is located on the driving roller. When each individual pole of the polar wheel passes by an angular sensor, a pulse is generated and supplied to the traveling time correlater to present a range for correct locking for an adjustment circuit. According to the Unexamined Japanese Patent Application Publication (Tokkai-Hei) No. 2002-348043, this configuration enables the length of the yarn defect to be accurately measured.

A yarn spun by the spinning machine may be periodically subjected to slight thickness unevenness. A possible cause of the periodic unevenness is, for example, decentering of a draft roller in the spinning machine which drafts a sliver. The periodic unevenness during the spinning step may cause moire in a woven cloth during a subsequent weaving step. Thus, the periodic unevenness needs to be detected and corrected earlier.

In general, to detect the above-described periodic yarn unevenness, frequency analysis is effectively used. A spinning machine with a constant yarn spinning speed can detect the periodic unevenness by allowing the yarn clearer to monitor the yarn traveling at the constant speed to obtain a yarn unevenness signal and performing a fast Fourier transform (FFT) operation on the yarn unevenness signal. Furthermore, a device called a yarn unevenness tester can also detect the periodic unevenness of the yarn by allowing a sensor to monitor a yarn being travelled at a constant speed, and performing spectrum analysis on the resulting signal. These detecting methods are effective when the yarn speed is constant.

For the frequency analysis, in general, a sampling frequency needs to have a limited value so as to set a frequency resolution to a fixed and appropriate rational number. The limited value of the sampling frequency allows the periodic unevenness to be sufficiently detected if the yarn speed is constant. However, for an automatic winder with the yarn speed varying as described above, a frequency at which a spectrum corresponding to the periodic unevenness appears also varies. Thus, the periodic unevenness cannot be detected by frequency analysis with the sampling frequency fixed. Consequently, the periodic unevenness or the like occurring in the spinning step conventionally fails to be accurately detected during a rewinding step of the automatic winder.

In this connection, the Unexamined Japanese Patent Application Publication (Tokkai-Hei) No. 2002-348043 only discloses that the automatic wider described therein is effective for increasing the accuracy of measurement of the yarn length (the length of the yarn unevenness), and fails to disclose the analysis of periodic unevenness in the yarn. Therefore, the conventional yarn clearer cannot accurately detect the periodic unevenness.

US 5 592 849 A discloses a yarn quality measuring instrument according to the preamble of claim 1.

### Summary of the Invention

The present invention has been made in view of the above-described circumstances. An object of the present invention is to provide a yarn quality measuring instrument and a yarn winder which can accurately evaluate the length of a yarn defect and detect the periodic unevenness.

An aspect of the present invention provides a yarn quality measuring instrument according to claim 1.

Thus, the sampling value can be obtained for every given yarn length regardless of the speed of the yarn. Consequently, yarn defects can be appropriately detected.

The yarn quality measuring instrument is configured as follows. That is, the yarn quality measuring instrument includes the first sensor, a second sensor, and the signal processing section. The first sensor detects the thickness of the traveling yarn. The second sensor is located at a predetermined distance from the first sensor on a yarn traveling path to detect the thickness of the traveling yarn. The signal processing section determines the yarn speed in accordance with signals from the first sensor and the second sensor, and samples the signal from the first sensor at the sampling frequency according to the yarn speed signal to detect the thickness unevenness in the yarn.

Thus, the yarn speed can be measured, and the sampling frequency can be varied according to the yarn speed. Furthermore, the signal processing section performs sampling for detecting the yarn speed, and concurrently performs sampling for measuring yarn quality. The signal processing section can thus accurately measure the yarn quality in real time. Moreover, the signal from the first sensor is used for both the detection of the yarn speed and the measurement of the yarn quality. A simple configuration can thus be provided.

In the yarn quality measuring instrument, the signal processing section preferably uses the yarn speed signal obtained from the external device when determining the yarn speed in accordance with the signals from the first sensor and the second sensor.

Thus, the yarn speed is determined utilizing information from a source located outside of the yarn quality measuring instrument. As a result, the yarn speed can be appropriately detected, and the yarn quality measuring instrument can also be simplified.

In the yarn quality measuring instrument, the signal processing section preferably performs an FFT calculation on the sampled signal from the first sensor to detect the thickness unevenness in the traveling yarn.

Thus, even with a variation in yarn speed, periodic unevenness in the yarn can be reliably and accurately detected.

In the yarn quality measuring instrument, preferably, before the FFT calculation, the signal from the first sensor is processed by a digital low pass filter.

Thus, the adverse effect of a possible aliasing phenomenon can be avoided to achieve accurate frequency analysis. Furthermore, the use of the digital low pass filter allows a cutoff frequency to be easily changed according to a variation in sampling frequency.

In the yarn quality measuring instrument, the detection of the thickness unevenness in the yarn preferably includes at least detection of periodic thickness unevenness in the yarn.

That is, the yarn quality measuring instrument according to the present invention varies the sampling speed according to the yarn speed. Thus, the yarn quality measuring instrument according to the present invention can detect the periodic thickness unevenness in the yarn, which cannot be detected by a configuration with a fixed sampling frequency.

According to another aspect of the present invention, a yarn winding machine according to claim 6 is provided.

Thus, the sampling value can be obtained for every given yarn length regardless of the speed of the yarn. Consequently, yarn defects can be appropriately detected.

The yarn winding machine further includes a package driving section and a yarn speed detecting section. The package driving section rotationally drives a package into which the yarn is wound. The yarn speed detecting section detects the yarn speed of the traveling yarn. The yarn quality measuring instrument includes a first sensor and a signal processing section. The first sensor detects the thickness of the traveling yarn. The signal processing section receives a yarn speed signal from the yarn speed detecting section and samples a signal from the first sensor at a sampling frequency according to the yarn speed signal to detect the thickness unevenness in the yarn.

Thus, the sampling frequency can be var i ed accord i ng to the yarn speed signal.

Consequently, the sampling value can be obtained for every given yarn length regardless of the yarn speed.

The yarn winding machine preferably further includes a speed instructing section outputting a rotation speed instruction signal to the package driving section.

Thus, the signal from the speed instructing section can be utilized to appropriately detect the yarn speed.

In the yarn winding machine, the yarn speed detecting section preferably includes a rotation speed detecting section detecting a rotation speed of the package driving section.

Thus, the yarn speed is determined utilizing information from the rotation speed detecting section detecting the rotation speed of the package driving section. Consequently, the yarn speed can be appropriately detected.

In the yarn winding machine, the yarn speed detecting section preferably further includes a speed sensor detecting the thickness of the traveling yarn to detect the yarn speed.

The provision of the sensor dedicated to the detection of the yarn speed enables the yarn speed to be accurately detected. As a result, the yarn speed and the sampling frequency can be associated with each other to maintain an appropriate sampling interval. Therefore, the yarn speed can be accurately detected.

In the yarn winding machine, the yarn quality measuring instrument further includes a second sensor. The second sensor is located at a predetermined distance from the first sensor on a yarn traveling path to detect the thickness of the traveling yarn. The signal processing section samples the signal from the first sensor at the sampling frequency according to the yarn speed signal and signals from the first sensor and the second sensor to detect the thickness unevenness in the yarn.

Thus, the signal processing section performs sampling for detecting the yarn speed, and concurrently performs sampling for measuring yarn quality. The signal processing section can thus accurately measure the yarn quality in real time. Moreover, the signal from the first sensor is used for both the detection of the yarn speed and the measurement of the yarn quality. A simple configuration can thus be provided.

In the yarn winding machine, the signal processing section preferably performs an FFT calculation on the sampled signal from the first sensor to detect the thickness unevenness in the traveling yarn.

Thus, even with a variation in yarn speed, periodic unevenness can be reliably and accurately detected.

In the yarn winding machine, preferably, before the FFT calculation, the signal from the first sensor is processed by a digital low pass filter.

Thus, the adverse effect of a possible aliasing phenomenon can be avoided to achieve accurate frequency analysis. Furthermore, the use of the digital low pass filter allows a cutoff frequency to be easily changed according to a variation in sampling frequency.

In the yarn winding machine, the detection of the thickness unevenness in the yarn preferably includes at least detection of periodic thickness unevenness in the yarn.

That is, the yarn quality measuring instrument according to the present invention varies the sampling speed according to the yarn speed. Thus, the yarn quality measuring instrument according to the present invention can detect the periodic thickness unevenness in the yarn, which cannot be detected by a configuration with a fixed sampling frequency. Thus, the yarn quality is more strictly inspected, thus enabling the quality of products to be improved.

Other features, elements, processes, steps, characteristics and advantages of the present invention will become more apparent from the following detailed description of preferred embodiments of the present invention with reference to the attached drawings.

### Brief Description of the Drawings

Figure 1 is a side view of a winder according to a first embodiment of the present invention.
Figure 2 is a front view showing a general configuration of the winder.
Figure 3 is a block diagram of a clearer.
Figure 4 is a conceptual drawing showing how a yarn speed is detected.
Figure 5 is a diagram illustrating how sampling is performed when a sampling frequency is varied in proportion to the yarn speed.
Figure 6 is a front view showing a general configuration of a winder according to a second embodiment of the present invention.
Figure 7 is a front view showing a general configuration of a winder according to a third embodiment of the present invention.

### Detailed Description of the Preferred Embodiments

Preferred embodiments of the present invention will be described below with reference to the drawings.

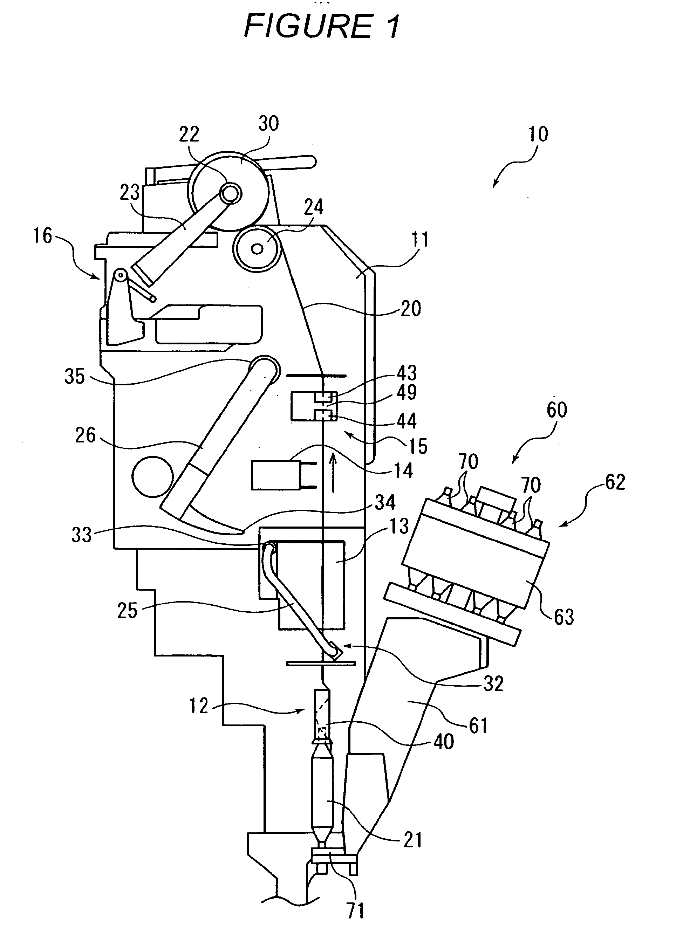

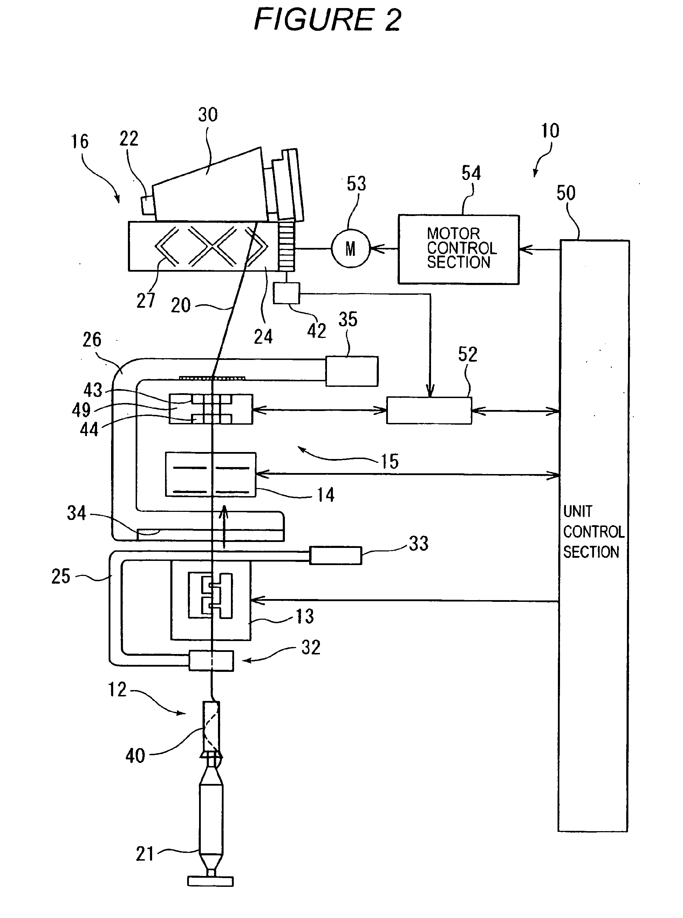

A winder unit 10 shown in Figures 1 and 2 winds a spun yarn 20 unwound from a yarn supplying bobbin 21 around a yarn winding bobbin 22 while traversing the spun yarn 20, to form a package 30 with a predetermined length and a predetermined shape. An automatic winder (yarn winding device) according to the present embodiment includes a plurality of the winder units 10 arranged in a line and a machine frame control device (not shown in the drawings) located at one end of the arrangement of the winder units 10 in a direction in which the winder units 10 are arranged.

Each of the winder units 10 includes a unit frame 11 (Figure 1) provided on one lateral side of the winder unit 10 in a front view, and a winding unit main body 16 provided on a side of the unit frame 11.

The winding unit main body 16 includes a cradle 23 and a winding drum (traverse drum) 24. The cradle 23 can grip a yarn winding bobbin 22. The winding drum 24 traverses the spun yarn 20, and drives the yarn winding bobbin 22. The cradle 23 is configured to be swingable in a direction in which the cradle 23 approaches or moves away from the winding drum 24. Thus, the cradle 23 allows the package 30 to make contact with or moves away from the winding drum 24. As shown in Figure 2, a spiral traverse groove 27 is formed on an outer peripheral surface of the winding drum 24. The spun yarn 20 is traversed by the traverse groove 27.

The cradle 23 includes a liftup mechanism and a package brake mechanism (neither of the mechanisms are shown in the drawings). When the yarn is broken, the liftup mechanism can elevate the cradle 23 to move the package 30 away from the winding drum 24. The package brake mechanism is configured to stop rotating the package 30 gripped by the cradle 23 at the same time when the cradle 23 is elevated by the liftup mechanism.

The winding unit main body 16 includes a balloon controller 12, a tensioning device 13, a splicer device (yarn splicing section) 14, and a clearer head 49 provided in a clearer (yarn quality measuring instrument) 15 arranged in this order from the yarn supplying bobbin 21 side in a yarn traveling route between the yarn supplying bobbin 21 and the winding drum 24.

As shown in Figure 1, the winder unit 10 includes a magazine-type supply device 60 that supplies the yarn supplying bobbin 21. The magazine-type supply device 60 includes a magazine holding section 61 and a bobbin housing device 62. The magazine holding section 61 extends obliquely forward and upward from a lower portion of the winder unit 10. The bobbin housing device 62 is attached to a tip of the magazine holding section 61.

The bobbin housing device 62 includes a magazine can 63. A plurality of housing holes are formed in the magazine can 63 and arranged in a circle. A supply bobbin 70 can be set in each of the housing holes in an inclined posture. The magazine can 63 can be intermittently driven and rotated by a motor (not shown in the drawings). This intermittent driving and a control valve (not shown in the drawings) provided in the magazine can 63 enable the supply bobbins 70 to be dropped one by one onto a bobbin supply path (not shown in the drawings) in the magazine holding section 61. The supply bobbin 70 supplied to the bobbin supply path is guided to a supplying bobbin holding section 71 while remaining in the inclined posture.

The supplying bobbin holding section 71 includes pivotally moving means (not shown in the drawings). Upon receiving the supply bobbin 70 via the bobbin supply path, the supplying bobbin holding section 71 pivotally moves the supply bobbin 70 so that the supplying bobbin 70 is raised from the oblique posture to an upright posture. Thus, the supply bobbin 70 is appropriately supplied to the lower portion of the winding unit main body 16 as a yarn supplying bobbin 21 so that the winder unit 10 can perform a winding operation. Alternatively, instead of the magazine-type supply device 60 as shown in Figure 1, a conveyor (not shown in the drawings) provided in a lower portion of the automatic winder may be used to feed the yarn supplying bobbin 21 from a supplying bobbin supply section (not shown in the drawings) to the supplying bobbin holding section 71 of each winder unit 10.

The balloon controller 12 lowers a regulating member 40 that covers a core tube of the yarn supplying bobbin 21, in conjunction with unwinding of the yarn from the yarn supplying bobbin 21. The balloon controller 12 thus assists in unwinding the yarn from the yarn supplying bobbin 21. The regulating member 40 contacts with a balloon formed above the yarn supplying bobbin 21 by the rotation and centrifugal force of the yarn unwound from the yarn supplying bobbin 21. The regulating member 40 thus applies an appropriate tension to the balloon to assist in unwinding the yarn. A sensor (not shown in the drawings) is provided in the vicinity of the regulating member 40 to detect a chase portion of the yarn supplying bobbin 21. When the sensor detects that the chase portion is being lowered, then the regulating member 40 can be lowered in conjunction with the lowering of the chase portion by an air cylinder (not shown in the drawings).

The tensioning device 13 applies a predetermined tension to the traveling spun yarn 20. The tensioning device 13 may be of, for example, a gate type having movable comb teeth arranged with respect to fixed comb teeth. The movable comb teeth can be pivotally moved by a rotary solenoid so as to be engaged with or released from the fixed comb teeth. The tensioning device 13 can apply a given tension to the yarn being wound, to improve the quality of the package 30. Besides the above-described gate type, for example, the tensioning device 13 of a disc type may be adopted.

When for example, the spun yarn 20 is cut because of a yarn defect detected by the clearer 15 or a yarn breakage occurs during unwinding of the spun yarn 20 from the yarn supplying bobbin 21, the splicer device 14 splices a lower yarn on the yarn supplying bobbin 21 side and an upper yarn on the package 30 side. For example, the splicer device 14 may be of a mechanical type or may use fluid such as compressed air.

The clearer 15 is configured to detect a defect by using an appropriate sensor to detect the thickness of the spun yarn 20. Specifically, two yarn unevenness sensors 43 and 44 are provided in the clearer head 49. The clearer 15 is configured so as to be able to detect a yarn defect such as a slab by allowing an analyzer 52 (Figure 2) to process signals from the two yarn unevenness sensors 43 and 44.

The clearer 15 can also function as a sensor that detects a traveling speed (yarn speed) of the spun yarn 20 and simply as a sensor that detects whether or not the spun yarn 20 is present. The clearer 15 will be described later in detail. A cutter (not shown in the drawings) is provided in the vicinity of the clearer head 49 to immediately cut the spun yarn 20 when the clearer 15 detects a yarn defect.

A lower yarn guide pipe 25 is provided below the splicer device 14 to catch and guide the lower yarn on the yarn supplying bobbin 21 side. An upper yarn guide pipe 26 is provided above the splicer device 14 to catch and guide the upper yarn on the package 30 side. A suction port 32 is formed at a tip of the lower yarn guide pipe 25. A suction mouth 34 is provided at a tip of the upper yarn guide pipe 26. An appropriate negative pressure source is connected to each of the lower yarn guide pipe 25 and the upper yarn guide pipe 26. Thus, suction flows can be generated at the suction port 32 and the suction mouth 34.

In this configuration, when yarn breakage or yarn cutting occurs, the suction port 32 of the lower yarn guide pipe 25 catches the lower yarn at a position shown in Figures 1 and 2. The lower yarn guide pipe 25 then pivotally moves upward around a shaft 33 to guide the lower yarn to the splicer device 14. Furthermore, almost at the same time, the upper yarn guide pipe 26 pivotally moves upward from an illustrated position around a shaft 35. The suction mouth 34 thus catches the upper yarn unwound from the package 30 rotated backward by a drum driving motor 53. The upper yarn guide pipe 26 subsequently moves pivotally downward around the shaft 35 to guide the upper yarn to the splicer device 14. Then, the splicer device 14 splices the lower yarn and the upper yarn.

The yarn unwound from the yarn supplying bobbin 21 is wound around the yarn winding bobbin 22, located on a downstream side of the splicer device 14. The yarn winding bobbin 22 is driven by rotationally driving the winding drum 24, located facing the yarn winding bobbin 22. As shown in Figure 2, the winding drum 24 is coupled to an output shaft of the drum driving motor (package driving section) 53. The operation of the drum driving motor 53 is controlled by a motor control section 54. The motor control section 54 controllably operates and stops the drum driving motor 53 in response to an operation signal from the unit control section 50.

A rotation sensor 42 is provided at the winding drum 24. The rotation sensor 42 is electrically connected to the analyzer 52 provided in the clearer 15. The rotation sensor 42 is configured as, for example, a rotary encoder. The rotation sensor 42 transmits a rotation pulse signal to the analyzer 52 every time the winding drum 24 rotates a predetermined angle. The analyzer 52 can acquire the rotation speed of the winding drum 24 (drum driving motor 53) by measuring the number of pulses per unit time.

In the above-described configuration, when the magazine-type supply device 60 supplies a bobbin to the bobbin holding section 71, the yarn winding bobbin 22 is driven. Then, the package 30 of a predetermined length can be formed by winding the spun yarn 20 unwound from the yarn supplying bobbin 21 around the yarn winding bobbin 22.

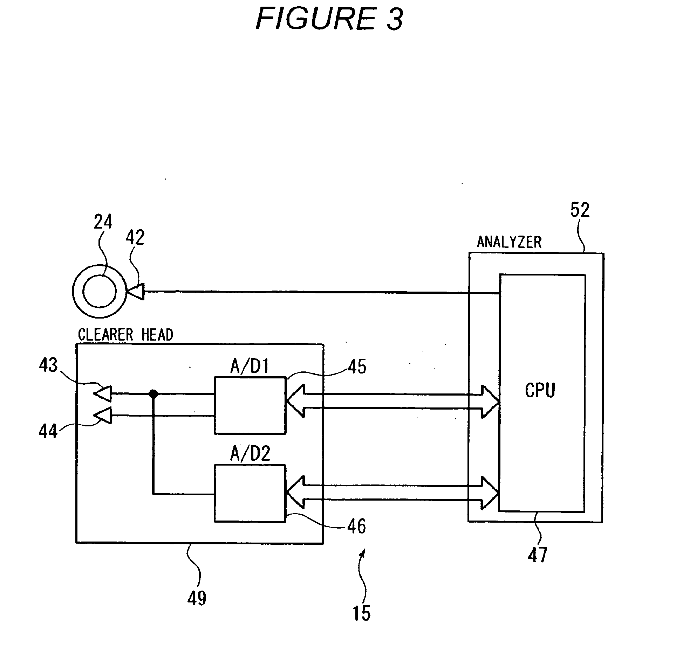

Next, the clearer 15 will be described with reference to Figure 3. As shown in Figure 3, the clearer head 49 includes the two yarn unevenness sensors 43 and 44, and two analog-to-digital (A/D) converters 45 and 46. Furthermore, the analyzer 52 includes a central processing unit (CPU) 47 as a control section and a signal processing section. Moreover, a pulse signal from the rotation sensor 42, which detects the rotation of the winding drum 24, is input to the analyzer 52.

The first yarn unevenness sensor 43 and the second yarn unevenness sensor 44 are arranged with an appropriate distance therebetween in a yarn traveling direction. In the yarn traveling direction, the first yarn unevenness sensor 43 is located on a downstream side and the second yarn unevenness sensor 44 is located on an upstream side.

The first A/D converter 45 samples analog signals from the two yarn unevenness sensors 43 and 44 to convert the sampled analog signals into digital signals. The resulting digital signals are input to the CPU 47, which uses the input digital signals to detect the yarn speed.

Concurrently with the sampling performed by the first A/D converter 45, the second A/D converter 46 samples the analog signal from the first yarn unevenness sensor 43 to convert the sampled analog signal into a digital signal. The resulting digital signal is input to the CPU 47, which uses the input digital signal to measure yarn quality.

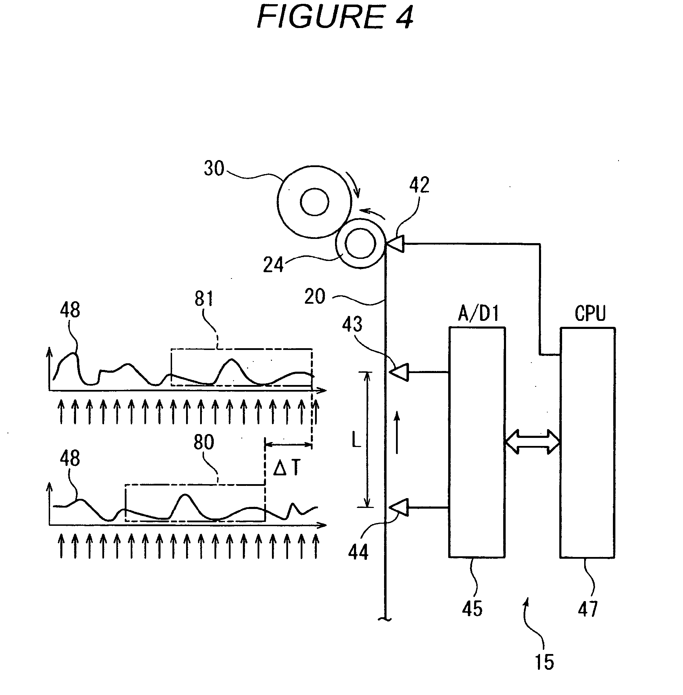

Next, with reference to Figure 4, a yarn speed detecting method performed by the clearer 15 will be described.

First, the first A/D converter 45 samples analog waveforms measured by the first yarn unevenness sensor 43 and the second yarn unevenness sensor 44. At this time, a sampling frequency fsl is set in proportion to the rotation speed of the winding drum 24. That is, the CPU 47 of the analyzer 52 calculates and acquires the rotation speed (drum rotation speed) of the winding drum 24 in accordance with a rotation detection signal for the winding drum 24 received from the rotation sensor 42 via a reception section (not shown in the drawings). The CPU 47 then multiplies the resulting drum rotation speed by a predetermined coefficient to determine the sampling frequency fsl. The CPU 47 then sets the determined sampling frequency fsl in the first A/D converter 45.

As described above, the CPU 47 utilizes the drum rotation speed as a rough indication of the yarn speed to vary the sampling frequency of the first A/D converter 45 according to the drum rotation speed. This enables the number of samples per unit yarn length to be maintained substantially constant when the signal waveforms of the first yarn unevenness sensor 43 and the second yarn unevenness sensor 44 are sampled by the first A/D converter 45. As a result, the yarn speed can be detected more accurately than when the sampling frequency is fixed.

In Figure 4, graphs shown to the left of the first yarn unevenness sensor 43 and the second yarn unevenness sensor 44 show examples of yarn unevenness signals provided by the first yarn unevenness sensor 43 and the second yarn unevenness sensor 44, respectively. The axis of abscissa indicates time, and the axis of ordinate indicates the intensity of the signal (for example, voltage). Upward arrows arranged in the time axis direction of each graph indicate sampling timings. A waveform 48 of a yarn unevenness signal is a continuous analog waveform obtained by measuring the thickness of the yarn. For example, a higher voltage is output when detecting thick yarn. A lower voltage is output when detecting thin yarn. However, what specific signal is output depends on the type of the sensor used.

Since the first yarn unevenness sensor 43 and the second yarn unevenness sensor 44 measure the same yarn, the same waveform 48 is observed. However, since the first yarn unevenness sensor 43 is located on the downstream side of the second yarn unevenness sensor 44 in the yarn traveling direction, the waveform detected by the first yarn unevenness sensor 43 is delayed compared with the waveform detected by the second yarn unevenness sensor 44.

Then, the CPU 47 compares data sampled by the first yarn unevenness sensor 43 with data sampled by the second yarn unevenness sensor 44. Specifically, an analysis frame 81 for a predetermined period of time at the current point of time is extracted from the waveform of the first yarn unevenness sensor 43.
Furthermore, from the waveform of the second yarn unevenness sensor 44, an analysis frame 80 for a predetermined period of time is extracted one after another from the waveform while gradually going back in time. The CPU 47 then compares the waveforms of the analysis frames 80 and 81 with each other. It is then assumed that as a result of repeated comparisons, the analysis frame 80 in the waveform of the second yarn unevenness sensor 44 obtained ΔT earlier than the current time matches the analysis frame 81 of the waveform of the first yarn unevenness sensor 43. In this case, when the distance between the first yarn unevenness sensor 43 and the second yarn unevenness sensor 44 is defined as L, the yarn speed V is determined by V = L/ΔT. As described above, the CPU 47 can accurately calculate the yarn speed V by executing a pattern matching process on the waveforms of the first yarn unevenness sensor 43 and the second yarn unevenness sensor 44.

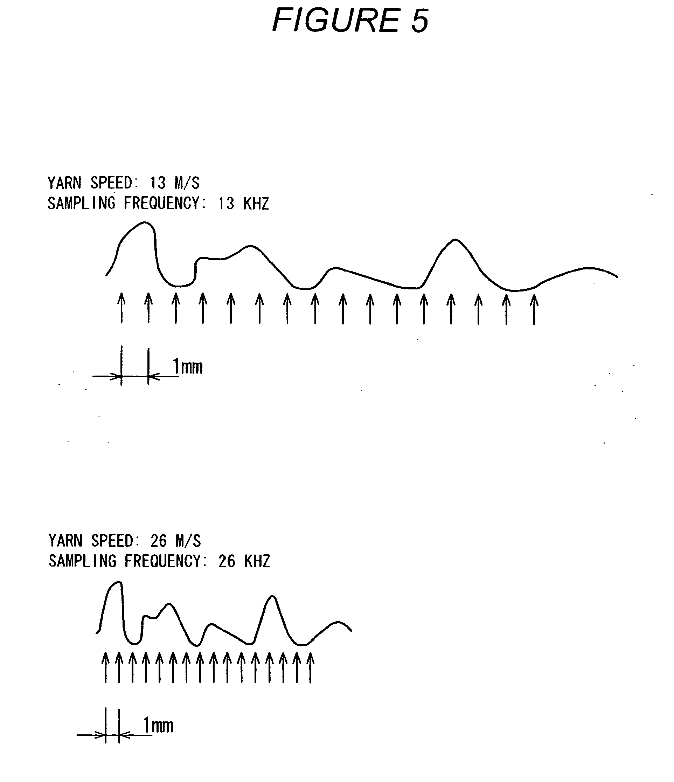

Next, sampling of the yarn unevenness signals for detection of a yarn defect will be described with reference to Figure 5. Figure 5 shows how the A/D converter 46 samples the yarn unevenness signal from the first yarn unevenness sensor 43.

Here, the sampling frequency of the second A/D converter 46 depends on a predetermined sample interval length, the yarn speed V obtained by the above-described process, and a predetermined oversampling scale.

The sample interval length is set to a sufficiently small value so as to detect frequency unevenness to be detected in an FFT calculation described below. That is, in accordance with a known Nyquist sampling theorem, the sample interval length is set to be at most half the period length of the periodic unevenness to be analyzed.

Furthermore, to prevent the adverse effect of loop-back (aliasing phenomenon) of a high frequency during FFT analysis, a process with a digital low pass filter is executed before the FFT calculation. The above-described oversampling scale is used to specify the scale of oversampling for the digital filter.

Thus, the sampling frequency fs2 of the second A/D converter 46 is determined by fs2 = yarn speed/(sample interval length/oversampling scale).

This will be described using specific numerical values. For example, it is assumed that the oversampling scale is 16 and that the sample interval length is 16 mm. In this case, the second A/D converter 46 performs sampling for every 1 mm of yarn length regardless of the yarn speed.

A waveform observed when the yarn speed is 13 m/s is shown in the upper part of Figure 5. A waveform observed when the yarn speed is 26 m/s is shown in the lower part of Figure 5. Since the yarn speed is higher in the lower part than in the upper part of Figure 5, the analog waveform detected by the first yarn unevenness sensor 43 is compressed in the time axis direction compared with the waveform shown in the upper part of Figure 5. However, in the present embodiment, the sampling frequency fs2 of the second A/D converter 46 varies in proportion to the accurate yarn speed V obtained through the above-described waveform delay analysis. The sampling frequency is set to 13 kHz in the example in the upper part of Figure 5 and to 26 kHz in the example in the lower part. Thus, the yarn length interval for the sampling is constant at 1 mm in both examples in the upper and lower parts of Figure 5, resulting in data of the same pattern. Thus, by setting the sampling frequency in proportion to the accurate yarn speed, the sampling can always be performed for every constant yarn length regardless of a variation in yarn speed.

Then, a frequency band is limited to at most a desired frequency in order to prevent the possible aliasing phenomenon. In the present embodiment, the digital low pass filter attenuates signal components with a cutoff frequency equal to or higher than a predetermined value. The use of the digital low pass filter allows the cutoff frequency to be easily varied according to a variation in sampling frequency resulting from a variation in yarn speed. Thus, spectrum analysis can be accurately performed.

After the above-described process is executed, the CPU 47 performs the known FFT calculation to detect the periodic unevenness. Furthermore, aperiodic yarn defects are detected. In this case, as described above, the yarn unevenness signals are always sampled at constant yarn length intervals regardless of the yarn speed. Thus, the spectrum of the periodic unevenness always appears at the same position, enabling the periodic unevenness to be accurately detected.
Furthermore, even for aperiodic, sporadic yarn defects, detection accuracy is improved because the length of the yarn unevenness can be accurately evaluated.

As described above, in the present embodiment, the winder unit 10, which constitutes the automatic winder, includes the clearer 15. The clearer 15 detects thickness unevenness in the spun yarn 20 traveling with the speed thereof varied. The clearer 15 allows the second A/D converter 46 to sample the detected yarn unevenness signal, and varies the sampling frequency fs2 of the second A/D converter 46 according to the yarn speed.

Thus, a sampling value for every given yarn length can be obtained regardless of the yarn speed. Consequently, yarn defects can be appropriately detected.

Furthermore, the clearer 15 includes the first yarn unevenness sensor 43, which detects the thickness of the traveling yarn, and the CPU 47 as a signal processing section. The CPU 47 receives a yarn speed signal obtained from the rotation sensor 42 as an external device for the clearer 15. The CPU 47 samples signals from the first yarn unevenness sensor 43 at the sampling frequency according to the yarn speed signal to detect thickness unevenness in the yarn.

Accordingly, since the sampling frequency can be varied according to the yarn speed signal, the sampling value for every given length can be obtained regardless of the yarn speed.

Furthermore, the clearer 15 includes the second yarn unevenness sensor 44. The second yarn unevenness sensor 44 is located at a predetermined distance from the first yarn unevenness sensor 43 on the yarn traveling route to detect the thickness of the traveling yarn. The CPU 47 determines the yarn speed in accordance with the yarn unevenness signals from the first yarn unevenness sensor 43 and the second yarn unevenness sensor 44. The CPU 47 samples the yarn unevenness signal from the first yarn unevenness sensor 43 at the sampling frequency corresponding to the yarn speed to detect thickness unevenness in the yarn.

Thus, by measuring the yarn speed and varying the sampling frequency of the second A/D converter 46 according to the yarn speed, the yarn quality can be appropriately measured using the second A/D converter 46. Furthermore, by allowing the first A/D converter 45 to perform the sampling for detecting the yarn speed and concurrently allowing the second A/D converter 46 to perform the sampling for measuring the yarn quality, the yarn quality can be accurately measured in real time. Moreover, the signal from the first yarn unevenness sensor 43 is used for both the detection of the yarn speed and the measurement of the yarn quality. The configuration of the clearer head 49 can be simplified.

Furthermore, in the clearer 15, in determining the yarn speed in accordance with the signals from the first yarn unevenness sensor 43 and the second yarn unevenness sensor 44, the CPU 47 uses the yarn speed signal obtained from the rotation sensor 42 as an external device.

Thus, the yarn speed is determined utilizing the information obtained from the source located outside the clearer 15. As a result, the yarn speed can be appropriately detected, and the clearer 15 can be simplified.

Furthermore, in the clearer 15, the CPU 47 carries out the FFT calculation on the sampled yarn unevenness signal from the first yarn unevenness sensor 43 to detect thickness unevenness in the traveling spun yarn 20.

Thus, even with a variation in yarn speed, the periodic unevenness can be accurately detected.

Furthermore, in the clearer 15, before the FFT calculation, the yarn unevenness signal from the first yarn unevenness sensor 43 is processed by the digital low pass filter.

Thus, frequency analysis can be accurately achieved. Furthermore, unlike when using an analog filter, the use of the digital low pass filter allows the cutoff frequency to be easily varied according to a variation in sampling frequency.

Furthermore, in the clearer 15, thickness unevenness in the yarn is detected by detecting periodic thickness unevenness in the yarn.

That is, the clearer 15 varies the sampling speed according to the yarn speed. Thus, the clearer 15 can detect the periodic thickness unevenness in the yarn, which cannot be detected by a configuration with a fixed sampling frequency.

Furthermore, the winder unit 10, which constitutes the automatic winder according to the present embodiment, includes the drum driving motor 53, which rotationally drives the package 30, and the yarn speed detecting section (rotation sensor 42) that detects the yarn speed of the traveling yarn. The CPU 47 as the signal processing section of the clearer 15 receives the yarn speed signal from the rotation sensor 42. The CPU 47 then samples the yarn unevenness signal from the first yarn unevenness sensor 43 at the sampling frequency according to the yarn speed signal to detect the thickness unevenness in the yarn.

Thus, during the detection of the yarn speed, the sampling period of the first A/D converter 45 can be associated with the rotation speed of the winding drum 24. As a result, an appropriate sampling interval can be maintained, and the yarn speed can be accurately detected.

Furthermore, in the present embodiment, the winder unit 10 includes the rotation sensor 42, which detects the rotation speed of the drum driving motor 53. The rotation sensor 42 functions as a yarn speed detecting section.

Thus, the rotation speed of the package driving section can be used as a yarn speed signal, enabling the appropriate sampling interval to be maintained. In the description of the present embodiment, the rotation sensor 42 is described as an example of the yarn speed detecting section. However, another sensor may be adopted provided that the sensor can detect the yarn speed of the traveling yarn.

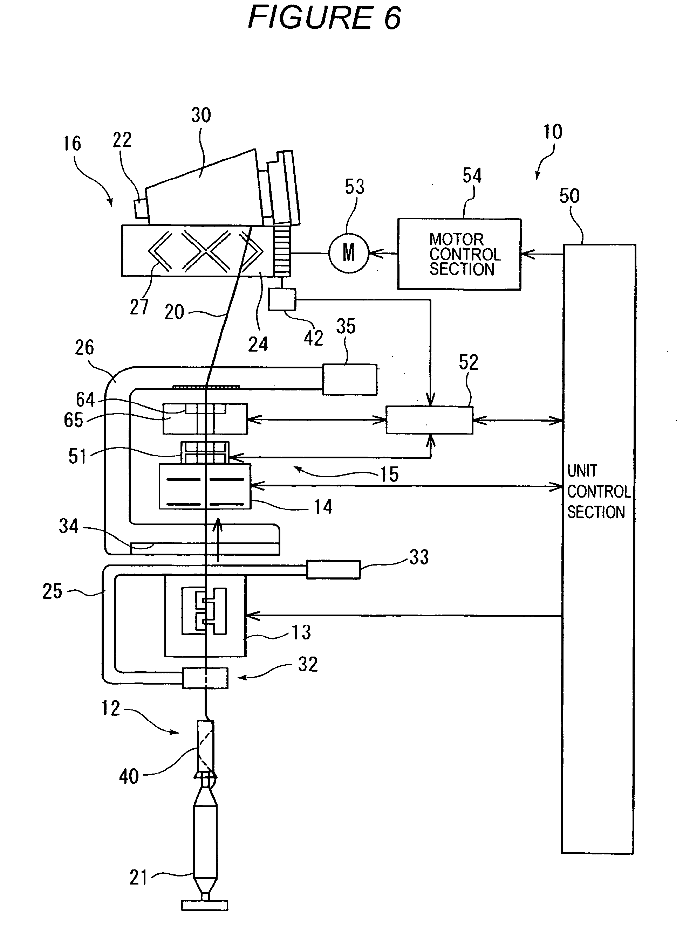

Next, a second embodiment of the present invention will be described with reference to Figure 6. In the present embodiment, a clearer head 65 in the clearer 15 has only one yarn unevenness sensor 64, and uses a dedicated speed sensor 51 to detect the yarn speed. Furthermore, in the present embodiment, only one A/D converter is required to sample the yarn unevenness signal.

The speed sensor 51 is a device that detects the yarn speed of the spun yarn 20 in a non-contact manner. The speed sensor 51 is configured to utilize a variation in yarn thickness to detect a moving speed of an area of the yarn which involves a variation in yarn thickness and to output a detected value.
Specifically, the speed sensor 51 includes a plurality of optical yarn thickness detecting sections arranged along the yarn traveling direction. The yarn speed sensor 51 detects the traveling speed of the spun yarn 20 in accordance with output signals from the yarn thickness detecting sections, which are arranged at different positions in the yarn traveling direction. Each of the optical yarn thickness detecting sections includes a light receiving element and a light source. The quantity of light received by the light receiving element varies depending on the thickness of the spun yarn 20 passing by a detection position of the yarn thickness detecting section.

A position where the speed sensor 51 is provided is not particularly limited. In the present embodiment, the speed sensor 51 is provided on the upstream side of the clearer 15 in the yarn traveling direction in an area that is suitable for detection of the yarn speed. For example, as shown in Figure 6, the speed sensor 51 is provided between the clearer 15 and the splicer device 14. Furthermore, in the present embodiment, the analyzer 52 calculates the drum rotation speed in accordance with the rotation speed signal from the rotation sensor 42. The speed sensor 51 varies a detection period of the yarn thickness detecting sections in proportion to the drum rotation speed. Thus, the yarn speed can be accurately detected as in the case of the determination of the yarn speed in the first embodiment.

The yarn speed detected value from the speed sensor 51 is input to the analyzer 52. The analyzer 52 varies the sampling frequency of the yarn unevenness sensor 64 in proportion to the yarn speed detected value.

As described above, the sampling value can also be obtained for every given yarn length by allowing the dedicated speed sensor 51 to detect the speed of the spun yarn 20 and varying the sampling frequency in proportion to the detected yarn speed. Thus, as is the case with the above-described embodiment, the periodic unevenness can be accurately detected by the FFT calculation. Furthermore, since only one yarn unevenness sensor is required, a clearer of an existing configuration can advantageously be used for the present embodiment. However, the method of detecting the yarn speed by comparing the yarn unevenness signals from the two yarn unevenness sensors as in the configuration in Figure 5 is more advantageous in the easiness of the incorporation of the speed sensor 51 into the clearer 15 and the ability to utilize the yarn unevenness signal directly for the detection of the yarn speed.

As described above, the winder unit 10, which constitutes the automatic winder in the embodiment shown in Figure 6, further includes the speed sensor 51, which detects the thickness of the traveling spun yarn 20 to detect the yarn speed. The speed sensor 51 functions as a yarn speed detecting section.

The provision of the speed sensor 51, which is dedicated to the detection of the yarn speed, enables the yarn speed to be accurately detected. As a result, the yarn speed can be associated with the sampling frequency to maintain the appropriate sampling interval. Therefore, the yarn speed can be accurately detected.

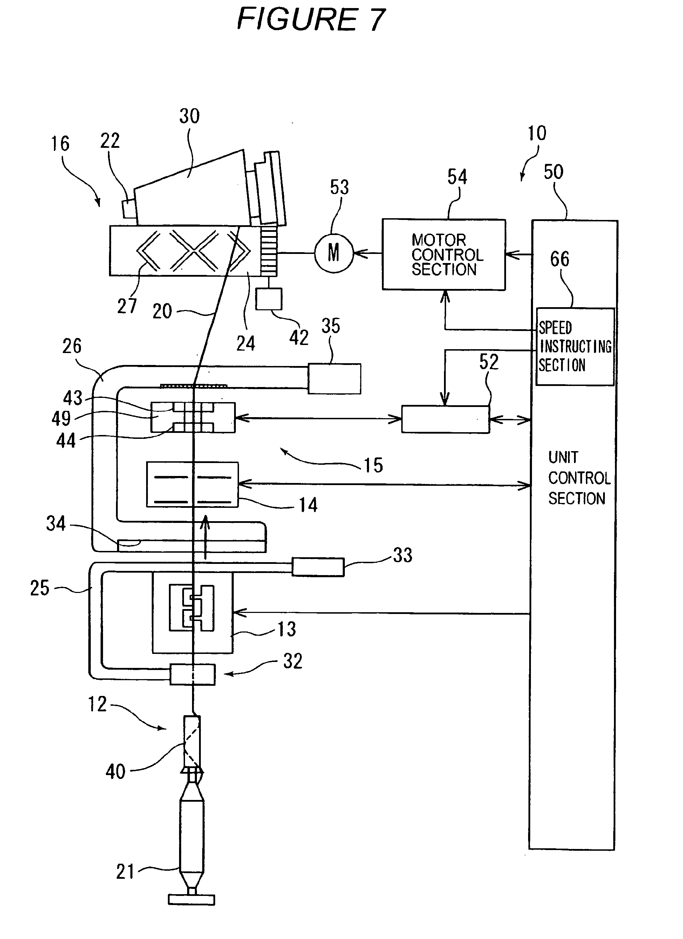

Next, a third embodiment of the present invention will be described with reference to Figure 7. In the present embodiment, a clearer head 49 includes the two yarn unevenness sensors 43 and 44 as is the case with the first embodiment.
Furthermore, the winder unit 10 includes a speed instructing section 66 that specifies a rotation speed for the drum rotation motor 53. The speed instructing section 66 receives yarn winding condition information (for example, winding speed and acceleration time) from the unit control section 50. The speed instructing section 66 then calculates the rotation speed of the winding drum 24 in accordance with the yarn winding condition information. The speed instructing section 66 then transmits a rotation speed instruction signal to the motor control section 54 to specify the rotation speed for the drum driving motor 53.

Furthermore, the rotation speed instruction signal is input to the analyzer 52. The analyzer 52 calculates and acquires the drum rotation speed from the rotation speed instruction signal. The analyzer 52 multiplies the drum rotation speed by a predetermined coefficient to determine the sampling frequency of the first A/D converter 45, and acquires the yarn speed by a method similar to that in the first embodiment. Thus, sampling can be performed at a sampling frequency proportional to the rotation speed of the winding drum 24, enabling the yarn speed to be accurately detected. By sampling the yarn unevenness signal so that the sampling frequency is proportional to the detected accurate yarn speed, periodic and aperiodic yarn unevenness can be accurately detected.

The third embodiment may be modified such that a speed sensor dedicated to detection of the yarn speed is provided and such that the clearer head includes only one yarn unevenness sensor. In this configuration, the analyzer 52 calculates and acquires the drum rotation speed in accordance with the rotation speed instruction signal from the speed instructing section 66. Then, the analyzer 52 transmits the acquired drum rotation speed to the speed sensor 51. The speed sensor 51 varies a detection period of the yarn thickness detecting section in proportion to the drum rotation speed. Accordingly, the yarn speed sensor 51 can accurately detect the yarn speed.

As described above, the winder unit 10, which constitutes the automatic winder in the embodiment shown in Figure 7, further includes the speed instructing section 66, which outputs the rotation speed instruction signal to the drum driving motor 53.

Thus, the signal from the speed instructing section 66 can be utilized to appropriately detect the yarn speed.

The preferred embodiments of the present invention have been described above.
However, the above-described configurations can be modified as follows.

In the above-described embodiments, both the detection of the periodic yarn unevenness and the detection of the aperiodic unevenness are performed. However, this configuration can be modified such that only the detection of the periodic yarn unevenness or the detection of the aperiodic unevenness is performed. That is, according to the embodiments of the present invention, since the sampling frequency of the yarn unevenness signal is varied according to the yarn speed, both the periodic yarn unevenness and the aperiodic unevenness can be accurately detected.

In the above-described embodiments, the control section and the signal processing section (CPU 47) provided in the analyzer 52 may be provided in the clearer head 49 or provided on the winder unit 10 side. Alternatively, a plurality of CPUs may be provided so as to separately execute the speed calculation, FFT calculation, and the low pass filter process. Moreover, the first A/D converter 45 and the second A/D converter 46 may be provided on the analyzer 52 side.

In the above-described embodiments, the sampling frequency of the first A/D converter 45 is varied in proportion to the rotation speed of the winding drum 24. However, this configuration is not necessarily indispensable, and the sampling may be performed at a fixed frequency. However, the sampling frequency is preferably varied using the rotation speed of the winding drum 24 as a rough indication of the yarn speed. This method allows the appropriate waveform to be acquired and subjected to the pattern matching process to achieve accurate speed analysis. Furthermore, the detection period of the yarn thickness detecting section of the speed sensor 51 may be fixed. However, also in this case, the speed can be accurately detected by using the rotation speed of the winding drum 24 as a rough indication of the yarn speed.

In addition, the configurations of the above-described embodiments may be applied to, for example, an automatic winder which does not use the winding drum (traverse drum) with the traverse groove 27 and which is configured such that the output shaft of the motor is coupled to the shaft portion (yarn winding bobbin 22) of the package 30 to drive the package 30 and a reciprocating traverse guide is used to traverse the yarn. In this case, a rotation sensor may be provided for detecting the rotation of a driving motor for the package 30 so that the yarn unevenness signals from the first yarn unevenness sensor 43 and the second yarn unevenness sensor 44 are sampled in accordance with signals from the rotation sensor (signals from the device located outside the clearer 15).

Instead of being configured as described above, the speed sensor may be modified to use another non-contact speed detecting method, for example, a method for detecting the yarn speed by a laser Doppler method or the like.

The low pass filter process may also be executed by an analog filter provided between the first yarn unevenness sensor 43 and the second yarn unevenness sensor 46. However, it is preferable to use the digital filter as described above in embodiments because the cutoff frequency can be quickly changed in response to a change in yarn speed.

The type of the first yarn unevenness sensor 43 and the second yarn unevenness sensor 44 may be changed. For example, optical sensors or electrostatic capacitance sensors may be used.

The description of the above-described embodiments relates to the automatic winder. However, the scope of the present invention is not limited to this aspect. The present invention is applicable to other winding machines, for example, spinning machines.

While the present invention has been described with respect to preferred embodiments thereof, it will be apparent to those skilled in the art that the disclosed invention may be modified in numerous ways and may assume many embodiments other than those specifically set out and described above. Accordingly, the appended claims cover all modifications of the present invention that fall within the scope of the invention.

## Claims

1. Yarn quality measuring instrument for detecting thickness unevenness in a yarn travelling with a varying speed by sampling the yarn unevenness detecting signal of the measuring instrument, adapted to vary the sampling frequency of the measuring instrument according to the varying speed of the yarn to detect the thickness unevenness in the yarn, **characterized by** including a first sensor (43) adapted to detect the thickness of the traveling yarn, a second sensor (44) located at a predetermined distance from the first sensor (43) on a yarn traveling path and adapted to detect the thickness of the traveling yarn, and a signal processing section (52), the signal processing section (52) adapted to determine the yarn speed in accordance with signals from the first sensor (43) and the second sensor (44), and to sample the signal from the first sensor (43) at the sampling frequency according to the yarn speed to detect the thickness unevenness In the yarn.

2. The yarn quality measuring instrument according to Claim 1, **characterized In that** the signal processing section (52) is adapted to use a yarn speed signal obtained from an external device (42) to determine the yarn speed in accordance with the signals from the first sensor (43) and the second sensor (44).

3. The yarn quality measuring instrument according to Claim 1 or 2, **characterized in that** the signal processing section (52) is adapted to perform a Fast Fourier Transform (FFT) calculation on the sampled signal from the first sensor (43) to detect the thickness unevenness in the traveling yarn.

4. The yarn quality measuring instrument according to Claim 3 **characterized in that** the signal processing section (52) is provided with a digital low pass filter arranged to process the signal from the first sensor (43) before the FFT calculation.

5. The yarn quality measuring instrument according to any one of Claims 1 to 4, **characterized in that** the signal processing section (52) includes at least means for detection of periodic thickness unevenness in the yarn.

6. A yarn winding machine **characterized by** including a yarn quality measuring instrument according to one of the claims 1 to 6.

7. The yarn winding machine according to Claim 6, **characterized by** including a package driving section (53) adapted to rotationally drive a package into which the yarn is wound, and a yarn speed detecting section detecting the yarn speed of the traveling yarn.

8. The yarn winding machine according to Claim 7, **characterized by** further including a speed instructing section (66) adapted to output a rotation speed instruction signal to the package driving section (53).

9. The yarn winding machine according to Claim 7, **characterized in that** the yarn speed detecting section includes a rotation speed detecting section (42) adapted to detect a rotation speed of the package driving section (53).

10. The yarn winding machine according to Claim 9, **characterized in that** the yarn speed detecting section further includes a speed sensor (43) adapted to detect the thickness of the traveling yarn to detect the yarn speed.

11. The yarn winding machine according to Claim 8 or Claim 9, **characterized in that** the yarn quality measuring instrument further includes a second sensor (44) located at a predetermined distance from the first sensor (43) on a yarn traveling path and adapted to detect the thickness of the traveling yarn, and the signal processing section (52) adapted to sample the signal from the first sensor (43) at the sampling frequency according to the yarn speed signal and signals from the first sensor (43) and the second sensor (44) to detect the thickness unevenness in the yarn.

12. The yarn winding machine according to any one of Claims 7 to 11, **characterized in that** the signal processing section (52) is provided with a FFT calculation means adapted to process the sampled signal from the first sensor (43) to detect the thickness unevenness in the traveling yarn.

13. The yarn winding machine according to Claim 12, **characterized in that** the signal processing section (52) is provided with a digital low pass filter arranged to process the signal from the first sensor (43) before the FFT calculation.

14. The yarn winding machine according to any one of Claims 6 to 13, **characterized in that** the signal processing section (52) includes at least means for the detection of periodic thickness unevenness in the yarn.

15. Method for detecting thickness unevenness in a yarn travelling with a varying speed by sampling a yarn unevenness detecting signal of a measuring instrument, with a step of varying a sampling frequency of the measuring instrument according to varying speed to detect the thickness unevenness in the yarn, **characterized by** the following steps:
- detecting the thickness of the traveling yarn with a first sensor (43) and a second sensor (44) located at a predetermined distance from the first sensor (43) on a yarn traveling path and sending signals corresponding to the yarn thickness to a signal processing section (52),
- determining the yarn speed in accordance with the signals from the first sensor (43) and the second sensor (44) in the signal processing section (52),
- sampling the signal from the first sensor (43) at a sampling frequency according to the yarn speed to detect the thickness unevenness in the yarn.

16. The method according to Claim 15 **characterized in that** the signal processing section (52) performs a Fast Fourier Transform (FFT) calculation on the sampled signal from the first sensor (43) to detect the thickness unevenness in the traveling yarn.

17. The method according to Claim 16 **characterized in that** before the FFT calculation the signal form the first sensor (43) is processed by a digital low pass filter.

18. The method according to any one of Claims15 to 17, **characterized in that** the detection of the thickness unevenness in the yarn includes at least detection of periodic thickness unevenness in the yarn.

## Patentansprüche

1. Messinstrument für die Garnqualität zum Erfassen einer Ungleichmäßigkeit der Dicke in einem mit variierender Geschwindigkeit laufenden Garn durch Abtastung des Garnungleichmäßigkeit-Erfassungssignals des Messinstruments, dafür ausgelegt, die Abtastfrequenz des Messinstruments gemäß der variierenden Geschwindigkeit des Garns zu verändern, um die Ungleichmä-βigkeit der Dicke im Garn zu erfassen, **dadurch gekennzeichnet, dass** es einen ersten Sensor (43), der zum Erfassen der Dicke des laufenden Garns ausgelegt ist, einen in einem vorgegebenen Abstand von dem ersten Sensor (43) an einem Garnlaufweg angeordneten zweiten Sensor (44), der dafür ausgelegt ist, die Dicke des laufenden Garns zu erfassen, und einen Signalverarbeitungsabschnitt (52) einschließt, welcher Signalverarbeitungsabschnitt (52) dafür ausgelegt ist, die Garngeschwindigkeit in Übereinstimmung mit Signalen von dem ersten Sensor (43) und dem zweiten Sensor (44) zu bestimmen und das Signal von dem ersten Sensor (43) mit der Abtastfrequenz gemäß der Garngeschwindigkeit abzutasten, um die Ungleichmäßigkeit der Dicke im Garn zu erfassen.

2. Messinstrument für die Garnqualität nach Anspruch 1, **dadurch gekennzeichnet, dass** der Signalverarbeitungsabschnitt (52) dafür ausgelegt ist, ein Garngeschwindigkeitssignal, das von einer externen Einrichtung (42) erhalten wird, für die Bestimmung der Garngeschwindigkeit in Übereinstimmung mit den Signalen von dem ersten Sensor (43) und dem zweiten Sensor (44) zu verwenden.

3. Messinstrument für die Garnqualität nach Anspruch 1 oder 2, **dadurch gekennzeichnet, dass** der Signalverarbeitungsabschnitt (52) dafür ausgelegt ist, eine schnelle Fouriertransformations-Berechnung (FFT) an dem abgetasteten Signal von dem ersten Sensor (43) durchzuführen, um die Ungleichmäßigkeit der Dicke in dem laufenden Garn zu erfassen.

4. Messinstrument für die Garnqualität nach Anspruch 3, **dadurch gekennzeichnet, dass** der Signalverarbeitungsabschnitt (52) mit einem digitalen Tiefpassfilter versehen ist, das dafür angeordnet ist, das Signal von dem ersten Sensor (43) vor der FFT-Berechnung zu verarbeiten.

5. Messinstrument für die Garnqualität nach einem der Ansprüche 1 bis 4, **dadurch gekennzeichnet, dass** der Signalverarbeitungsabschnitt (52) zumindest Mittel zum Erfassen von periodischen Ungleichmäßigkeiten der Dicke im Garn aufweist.

6. Garnspulmaschine, **dadurch gekennzeichnet, dass** sie ein Messinstrument für die Garnqualität nach einem der Ansprüche 1 bis 5 enthält.

7. Garnspulmaschine nach Anspruch 6, **dadurch gekennzeichnet, dass** sie einen Auflaufspulenantriebsabschnitt (53), der dafür ausgelegt ist, eine Auflaufspule drehend anzutreiben, auf welche das Garn gewickelt wird, sowie einen Garngeschwindigkeitserfassungsabschnitt enthält, der die Garngeschwindigkeit des laufenden Garns erfasst.

8. Garnspulmaschine nach Anspruch 7, **dadurch gekennzeichnet, dass** sie ferner einen Geschwindigkeitsbefehlsabschnitt (66) aufweist, der dafür ausgelegt ist, ein Drehgeschwindigkeits-Befehlssignal an den Auflaufspulenantriebsabschnitt (53) auszugeben.

9. Garnspulmaschine nach Anspruch 7, **dadurch gekennzeichnet, dass** der Garngeschwindigkeitserfassungsabschnitt einen Drehgeschwindigkeitserfassungsabschnitt (42) aufweist, der dafür ausgelegt ist, eine Drehgeschwindigkeit des Auflaufspulenantriebsabschnitts (53) zu erfassen.

10. Garnspulmaschine nach Anspruch 9, **dadurch gekennzeichnet, dass** der Garngeschwindigkeitserfassungsabschnitt ferner einen Geschwindigkeitssensor (43) aufweist, der dafür ausgelegt ist, die Dicke des laufenden Garns zu erfassen, um die Garngeschwindigkeit zu erfassen.

11. Garnspulmaschine nach Anspruch 8 oder Anspruch 9, **dadurch gekennzeichnet, dass** das Messinstrument für die Garnqualität ferner einen zweiten Sensor (44) aufweist, der in einem vorbestimmten Abstand von dem ersten Sensor (43) an einem Garnlaufweg angeordnet ist und dafür ausgelegt ist, die Dicke des laufenden Garns zu erfassen, und der Signalverarbeitungsabschnitt (52) dafür ausgelegt ist, das Signal von dem ersten Sensor (43) mit der dem Garngeschwindigkeitssignal entsprechenden Abtastfrequenz und Signale von dem ersten Sensor (43) und dem zweiten Sensor (44) abzutasten, um Ungleichmäßigkeiten der Dicke im Garn zu erfassen.

12. Garnspulmaschine nach einem der Ansprüche 7 bis 11, **dadurch gekennzeichnet, dass** der Signalverarbeitungsabschnitt (52) mit einer FFT-Berechnungseinrichtung ausgerüstet ist, die dafür ausgelegt ist, das abgetastete Signal von dem ersten Sensor (43) zu verarbeiten, um die Ungleichmä-βigkeit der Dicke im laufenden Garn zu erfassen.

13. Garnspulmaschine nach Anspruch 12, **dadurch gekennzeichnet, dass** der Signalverarbeitungsabschnitt (52) mit einem digitalen Tiefpassfilter versehen ist, das dafür angeordnet ist, das Signal von dem ersten Sensor (43) vor der FFT-Berechnung zu verarbeiten.

14. Garnspulmaschine nach einem der Ansprüche 6 bis 13, **dadurch gekennzeichnet, dass** der Signalverarbeitungsabschnitt (52) zumindest Mittel zum Erfassen von periodischen Ungleichmäßigkeiten der Dicke im Garn aufweist.

15. Verfahren zum Erfassen von Ungleichmäßigkeiten der Dicke in einem mit einer variierenden Geschwindigkeit laufenden Garn durch Abtastung eines Garnungleichmäßigkeit-Erfassungssignals eines Messinstruments mit einem Schritt des Veränderns einer Abtastfrequenz des Messinstruments gemäß einer variierenden Geschwindigkeit, um die Ungleichmäßigkeit der Dicke im Garn zu erfassen, **gekennzeichnet durch** die folgenden Schritte:
- Erfassen der Dicke des laufenden Garns mit einem ersten Sensor (43) und einem zweiten Sensor (44), der in einem vorbestimmten Abstand von dem ersten Sensor (43) an einem Garnlaufweg angeordnet ist, und Senden von der Dicke entsprechenden Signalen zu einem Signalverarbeitungsabschnitt (52),
- Bestimmen der Garngeschwindigkeit in Übereinstimmung mit den Signalen von dem ersten Sensor (43) und dem zweiten Sensor (44) in dem Signalverarbeitungsabschnitt (52),
- Abtasten des Signals von dem ersten Sensor (43) mit einer der Garngeschwindigkeit entsprechenden Abtastfrequenz, um die Ungleichmäßigkeit der Dicke im Garn zu erfassen.

16. Verfahren nach Anspruch 15, **dadurch gekennzeichnet, dass** der Signalverarbeitungsabschnitt (52) eine schnelle Fouriertransformations-Berechnung (FFT) an dem abgetasteten Signal von dem ersten Sensor (43) durchführt, um die Ungleichmäßigkeit der Dicke im laufenden Garn zu erfassen.

17. Verfahren nach Anspruch 16, **dadurch gekennzeichnet, dass** das Signal von dem ersten Sensor (43) vor der FFT-Berechnung von einem digitalen Tiefpassfilter verarbeitet wird.

18. Verfahren nach einem der Ansprüche 15 bis 17, **dadurch gekennzeichnet, dass** die Erfassung der Ungleichmäßigkeit der Dicke im Garn zumindest die Erfassung einer periodischen Ungleichmäßigkeit der Dicke im Garn einschließt.

## Revendications

1. Instrument de mesure de la qualité d'un fil pour détecter une inégalité d'épaisseur dans un fil circulant à vitesse variable en échantillonnant le signal de détection de l'inégalité du fil de l'instrument de mesure, qui est à même de faire varier la fréquence d'échantillonnage de l'instrument de mesure selon la vitesse variable du fil pour détecter l'inégalité d'épaisseur dans celui-ci, **caractérisé en ce qu'**il comprend un premier capteur (43) qui est à même de détecter l'épaisseur du fil circulant, un second capteur (44) situé à une distance prédéterminée du premier capteur (43) sur un trajet de circulation de fil et qui est à même de détecter l'épaisseur du fil circulant, et une section de traitement de signal (52), la section de traitement de signal (52) étant à même de déterminer la vitesse du fil conformément à des signaux provenant du premier capteur (43) et du second capteur (44), et d'échantillonner le signal provenant du premier capteur (43) à la fréquence d'échantillonnage selon la vitesse du fil pour détecter l'inégalité d'épaisseur dans celui-ci.

2. Instrument de mesure de la qualité d'un fil selon la revendication 1, **caractérisé en ce que** la section de traitement de signal (52) est à même d'utiliser un signal de vitesse de fil tiré d'un dispositif externe (42) pour déterminer la vitesse du fil conformément aux signaux provenant du premier capteur (43) et du second capteur (44).

3. Instrument de mesure de la qualité d'un fil selon la revendication 1 ou la revendication 2, **caractérisé en ce que** la section de traitement de signal (52) est à même d'effectuer un calcul de la transformation accélérée de Fourier (FFT) sur le signal échantillonné provenant du premier capteur (43) pour détecter l'inégalité d'épaisseur dans le fil circulant.

4. Instrument de mesure de la qualité d'un fil selon la revendication 3, **caractérisé en ce que** la section de traitement de signal (52) est pourvue d'un filtre passe-bas numérique aménagé pour traiter le signal provenant du premier capteur (43) avant le calcul de la FFT.

5. Instrument de mesure de la qualité d'un fil selon l'une quelconque des revendications 1 à 4, **caractérisé en ce que** la section de traitement de signal (52) comprend au moins des moyens pour la détection de l'inégalité d'épaisseur périodique dans le fil.

6. Machine de bobinage de fil, **caractérisée en ce qu'**elle comprend un instrument de mesure de la qualité du fil selon l'une quelconque des revendications 1 à 5.

7. Machine de bobinage de fil selon la revendication 6, **caractérisée en ce qu'**elle comprend une section d'entraînement de bobine (53) qui est à même d'entraîner en rotation une bobine dans laquelle le fil est enroulé et une section de détection de la vitesse du fil détectant la vitesse du fil circulant.

8. Machine de bobinage de fil selon la revendication 7, **caractérisée en ce qu'**elle comprend en outre une section (66) d'instruction en matière de vitesse, qui est à même de délivrer un signal d'instruction en matière de vitesse de rotation à la section d'entraînement de bobine (53).

9. Machine de bobinage de fil selon la revendication 7, **caractérisée en ce que** la section de détection de vitesse du fil comprend une section de détection de vitesse de rotation (42) qui est à même de détecter une vitesse de rotation de la section d'entraînement de bobine (53).

10. Machine de bobinage de fil selon la revendication 9, **caractérisée en ce que** la section de détection de vitesse du fil comprend en outre un capteur de vitesse (43) qui est à même de détecter l'épaisseur du fil circulant pour détecter la vitesse de celui-ci.

11. Machine de bobinage de fil selon la revendication 8 ou la revendication 9, **caractérisée en ce que** l'instrument de mesure de la qualité du fil comprend en outre un second capteur (44) situé à une distance prédéterminée du premier capteur (43) sur un trajet de circulation du fil et qui est à même de détecter l'épaisseur du fil circulant, et la section de traitement de signal (52) est à même d'échantillonner le signal provenant du premier capteur (43) à la fréquence d'échantillonnage selon le signal de vitesse du fil et les signaux provenant du premier capteur (43) et du second capteur (44) pour détecter l'inégalité d'épaisseur dans le fil.

12. Machine de bobinage de fil selon l'une quelconque des revendications 7 à 11, **caractérisée en ce que** la section de traitement de signal (52) est pourvue d'un moyen de calcul de la FFT qui est à même de traiter le signal échantillonné provenant du premier capteur (43) pour détecter l'inégalité d'épaisseur dans le fil circulant.

13. Machine de bobinage de fil selon la revendication 12, **caractérisée en ce que** la section de traitement de signal (52) est pourvue d'un filtre passe-bas numérique aménagé pour traiter le signal provenant du premier capteur (43) avant le calcul de la FFT.

14. Machine de bobinage selon l'une quelconque des revendications 6 à 13, **caractérisée en ce que** la section de traitement de signal (52) comprend au moins des moyens pour la détection de l'inégalité d'épaisseur périodique dans le fil.

15. Procédé de détection de l'inégalité d'épaisseur dans un fil circulant à une vitesse variable en échantillonnant un signal de détection d'inégalité de fil d'un instrument de mesure, avec une étape visant à faire varier la fréquence d'échantillonnage de l'instrument de mesure selon la vitesse variable pour détecter l'inégalité d'épaisseur dans le fil, **caractérisé par** les étapes suivantes consistant à :
- détecter l'épaisseur du fil circulant avec un premier capteur (43) et un second capteur (44) situé à une distance prédéterminée du premier capteur (43) sur un trajet de circulation du fil et envoyer des signaux correspondant à l'épaisseur du fil à une section de traitement de signal (52),
- déterminer la vitesse du fil conformément aux signaux provenant du premier capteur (43) et du second capteur (44) dans la section de traitement de signal (52), et
- échantillonner le signal provenant du premier capteur (43) à une fréquence d'échantillonnage conformément à la vitesse du fil pour détecter l'inégalité d'épaisseur dans celui-ci.

16. Procédé selon la revendication 15, **caractérisé en ce que** la section de traitement de signal (52) effectue un calcul de la transformation accélérée de Fourier (FFT) sur le signal échantillonné provenant du premier capteur (43) pour détecter l'inégalité d'épaisseur dans le fil circulant.

17. Procédé selon la revendication 16, **caractérisé en ce que**, avant le calcul de la FFT, le signal provenant du premier capteur (43) est traité par un filtre passe-bas numérique.

18. Procédé selon l'une quelconque des revendications 15 à 17, **caractérisé en ce que** la détection de l'inégalité d'épaisseur du fil comprend au moins la détection de l'inégalité d'épaisseur périodique dans le fil.
